# EUROPEAN PATENT APPLICATION

(11) **EP 0 723 951 A1**
(43) Date of publication of application: **31.07.1996**
(21) Application number: 96830027.7
(22) Date of filing: 24.01.1996
(51) Int. Cl.: C07C 67/03, C07C 67/52, C07C 69/82

(54) **Process to prepare bis (2-hydroxyethyl) terephthalate**

(30) Priority: 24.01.1995 IT BO950017
(71) Applicant: ARS ING S.r.L., 10100 Torino (IT)
(72) Inventor: Pilati, Francesco, Bologna (IT); Toselli, Maurizio, Bologna (IT); Stramigioli, Carlo, Bologna (IT); Baldi, Giancarlo, Torino (IT); Capra, Massimo, Torino (IT); Osella, Mauro, Torino (IT); Bava Pilone, Gloria, Torino (IT)
(74) Representative: Coppi, Cecilia

(57) **Abstract**

This invention refers to a process to prepare very high purity bis(2-hydroxyethyl) terephthalate, using waste polyethylene terephthalate (PET) as the starting product. More specifically, the process is used to prepare BHET through the reaction of 5 waste PET with excess ethylene glycol (EG), and preferably at a temperature of 190-210°C, in the presence of a transesterification catalyzer. This is followed by a series of separations and crystallisations (for which three different methods are proposed) to obtain a pure, chemically defined 10 product that does not contain any of the impurities that may be found in the starting PET.

## Description

This invention refers to a process to prepare bis(2-hydroxyethyl) terephthalate (or bis-hydroxyethyl terephthalate or bis ethylene glycol terephthalate) hereinafter indicated by the acronym BHET.

More specifically, this invention refers to a process to prepare very high purity BHET using waste polyethylene terephthalate (PET) as the starting product which is recovered after use or comes from industrial wastes.

In addition to PET, this process uses other non-toxic products, ethylene glycol and water, and therefore is an alternative to the existing methods to recycle PET wastes.

The BHET produced with this process can be used by itself, or together with other monomers, to prepare new PET or other copolyesters or to produce resins for paints. In particular, because of the high purity of the BHET produced in this manner, it can be used to produce PET that is also suitable for food applications.

Typically, the PET wastes can be recycled using one of the following methods:
i) acidic or basic hydrolysis;
ii) methanolysis;
iii) glycolysis.

Method (i) uses water as the reagent together with PET and can be used to obtain terephthalic acid (TA) and ethylene glycol (EG) as reaction products (U.S. Patent 3,501,420, 1970). However, the reaction occurs only at high temperatures in the presence of strong concentrations of powerful acids or bases. These processes use reactors made with special materials that can withstand such severe conditions and also include a large number of operations (neutralisation, acidification, filtering, etc.) to recover the monomers, terephthalic acid and ethylene glycol. Furthermore, there are high quantities of inorganic by-products which must be properly eliminated. All this involves high investment and management costs.

Method (ii) uses methyl alcohol as a reagent that converts PET into a dimethyl terephthalate (DMT) monomer and ethylene glycol. This type of process has been widely studied and the technology for such a process has been developed, however PET reacts with methyl alcohol, in the presence of catalyzers, only at high temperatures (220-250 °C) and pressures (U.S. Patent 3,403,115, 1968). This involves risks regarding the use of methyl alcohol under such drastic conditions. In fact, it is well known that methyl alcohol is highly explosive at high temperatures and pressures. In addition, methyl alcohol is toxic if inhaled or if it comes in contact with the skin, eyes, etc.

In method (iii) waste PET reacts with ethylene glycol with the formation of a mix of PET oligomers that then can be used directly, without any purification, to produce new PET (U.S. Patent 4,609,680, 1986; U.S. Patent 3,222,299, 1965). This method requires relatively mild reaction conditions (220 °C, atmospheric pressure, use of EG) but, since the products obtained are not purified, it cannot eliminate the impurities and contamination (for example catalytic residues, dyes, various additives, diethylene glycol, etc.) found in the waste PET used. As a consequence, the quality of the PET that can be obtained with this type of process will be lower and, for example, is not suitable for food applications.

The process proposed in this paper is not as complex as the one indicated in point (i), the conditions are not as risky compared to those in process (ii) and products can be obtained that have a higher quality than those obtained with process (iii). More specifically, the process described in this invention has the following advantages:
- No hazardous or toxic products;
- The BHET obtained can be polymerised as such to prepare PET, eliminating the first stage of the process and the relative catalyzers;
- The BHET obtained can be added to the mix of oligomers resulting from the first stage of polymerisation of the traditional PET polymerisation processes, starting from either terephthalic acid or from DMT;
- BHET is a pure, chemically well-defined product, without any of the impurities that may be contained in the starting PET. In addition, PET can be prepared, using BHET as a monomer, in a single stage, and therefore using systems that are simpler than the traditional methods (in two stages). Thus, it is possible to eliminate the reactor of the first stage, to use only antimony salts as catalyzers and to avoid adding the substances that are usually added to cancel the negative effects of the first stage catalyzers. Therefore, the product obtained will have better characteristics compared to those which can be obtained, for example, with processes (ii) and (iii). In particular, such a product will have greater heat and hydrolytic stability, less possibility of transfer phenomena and improved electrical properties.

In the process proposed in this invention to prepare BHET, waste PET reacts with excess ethylene glycol in the presence of a transesterification catalyzer and the BHET is recovered through crystallisation from an aqueous solution.

The catalyzers that can be used for the first stage are those commonly utilised for the transesterification stage in PET polymerisation. Table 1 reports the relative speed of the depolymerization reaction in the presence of a series of catalyzers and the effects of their concentration and temperature.

**table 1.**

| Effect of the type and concentration of catalyzers and temperature on the speed of the 1st stage of the process. | | | | | |
|---|---|---|---|---|---|
| PET | EG | catalyzer | | Temperature | relative speed* |
| (g) | (ml) | type | mmol/kg (PET+EG) | (°C) | |
| 30 | 100 | Ti(OBu)₄ | 3.0 | 190 | 85 |
| 30 | 100 | Sb₂O₃ | 3.0 | 190 | <10 |
| 30 | 100 | CeAc₃ | 3.0 | 190 | 90 |
| 30 | 100 | LaAc₃ | 3.0 | 190 | 90 |
| 30 | 100 | GeO₂ | 3.0 | 190 | <10 |
| 30 | 100 | ZnAc₂ | 3.0 | 190 | 100 |
| 30 | 100 | CaAc₂ | 3.0 | 190 | 40 |
| 30 | 100 | CoAc₂ | 3.0 | 190 | 70 |
| 30 | 100 | CoAc₂ | 4.5 | 190 | 80 |
| 30 | 200 | CoAc₂ | 2.5 | 190 | 60 |
| 30 | 300 | CoAc₂ | 1.5 | 190 | 40 |
| 30 | 100 | CoAc₂ | 2.5 | 190 | 65 |
| 300 | 100 | CoAc₂ | 2.5 | 210 | 160 |
| 300 | 100 | CoAc₂ | 2.5 | 220 | 280 |

| | | | | | |
|---|---|---|---|---|---|
| * Corresponding to a conversion at BHET ≥ 90% | | | | | |

Therefore, the preferred catalyzers are the Zn, La and Ce acetates and Ti(Obu)₄. They are added to the first stage reactor, dissolved or dispersed in a small amount of boiling ethylene glycol, in such a quantity to obtain an overall concentration in the reactor of 0.2-1.5 and preferably 0.5 g/ kg of mix (PET+EG).

For both methods, the reactor can be fed either by adding PET and EG to the reactor, heating the EG before adding the PET, or by adding PET and EG together to the reactor that already contains a predetermined quantity of reaction mix at the required temperature. Ideal conditions for the first stage are an EG/ PET weight ratio between 1 and 5, preferably 2 and 3. For the case in which PET and EG are added to a reaction mix that is already present, the weight ratio between the overall amount added (PET+EG) and the mix already present in the reactor may vary from 0.2 to 5.

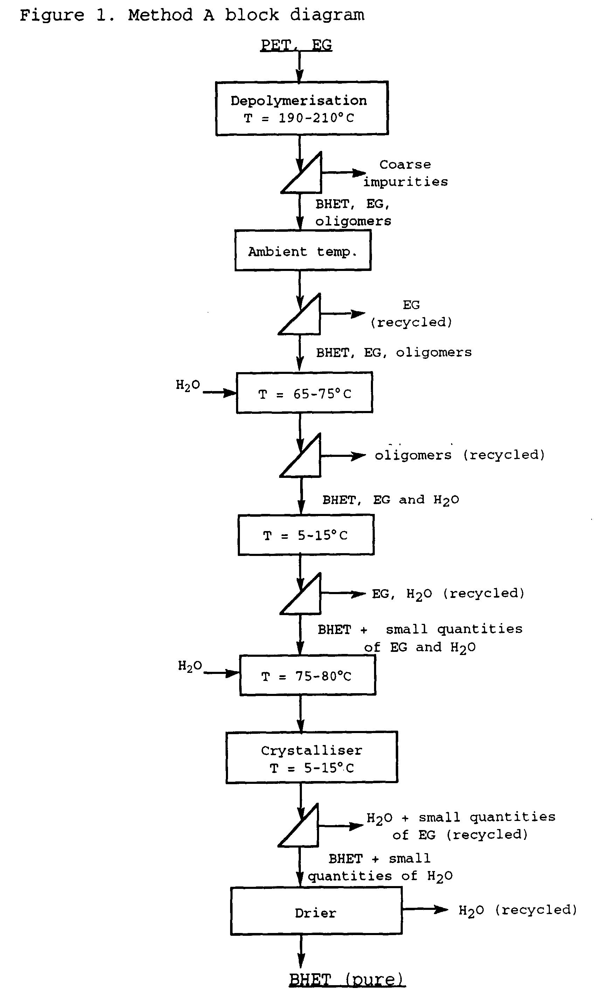

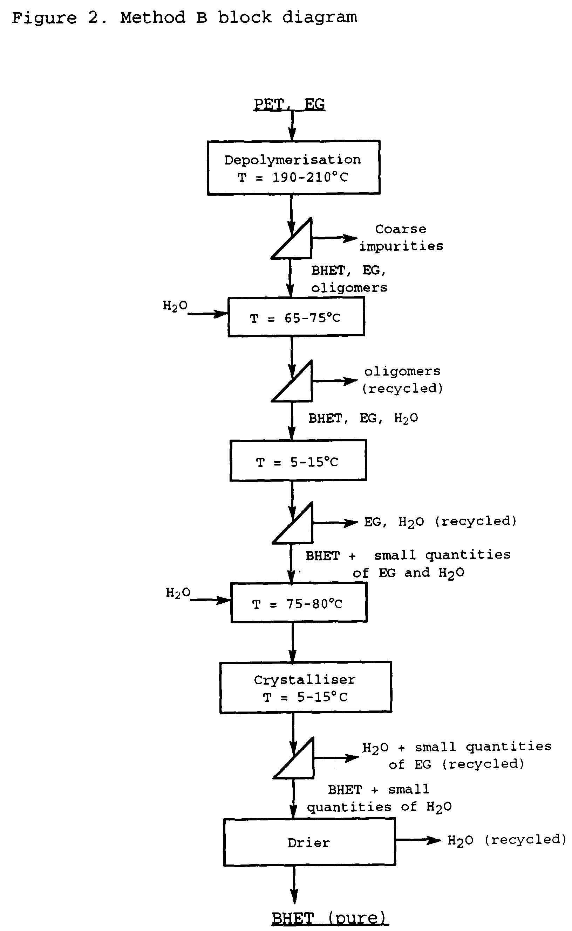

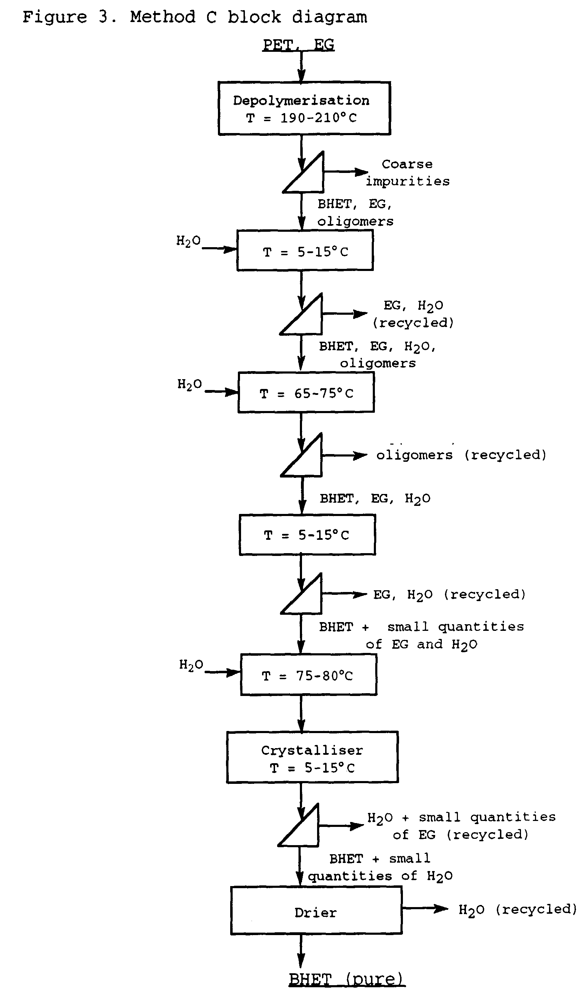

The process described in this invention is performed preferably according to three possible methods, (A) (B) and (C), which are reported schematically in figures 1, 2 and 3.

According to method (A) (figure 1), waste PET, EG (some coming from internal recycling and some as a virgin monomer) and the catalyzer are placed in the first reactor.

This first stage of the reaction is carried out at a temperature ranging from 180 to 270°C, and preferably between 190 and 210°C.

At the end of the first stage, the reaction mix is first filtered to eliminate any coarse impurities, then cooled at ambient temperature. A solid impregnated with liquid is separated out. This solid has a consistency of a viscous paste, from which a clear liquid can be separated through filtering or centrifuging. The liquid, containing most of the excess EG, is again placed in the reactor, while hot water is added to the solid in a quantity that may vary with respect to the filtering residue in a ratio from 1 to 10 (mass/mass), and preferably between 2 and 5, so that the temperature of the overall mix is 60-90°C, and preferably 65-75°C. The temperature control during this phase is particularly critical since, during this phase, the BHET must solubilize in the aqueous solution, while the oligomers, which otherwise co-crystallise with the BHET, preventing the formation of sufficiently large BHET crystals and contaminating it, must remain in suspension (see example 4).

After filtering, with separation of the oligomers, the aqueous solution is cooled slowly to precipitate the BHET, the water solubility of which varies according to the temperature as shown in graph 1 (see example 5). To obtain a high yield of BHET, the final temperature must reach -10/+30°C, and preferably 5-15°C. The BHET crystals are separated from the aqueous solution, which contains most of the excess EG, through filtering or centrifuging.

The solid recovered is dissolved again in hot water until reaching a temperature of 70-100°C and then cooled to 0-30°C, and preferably 5-15°C, to obtain high-purity BHET crystals that are easy to filter. In this second crystallisation phase, the ratio of the amount of water to BHET ranges between 4 and 10, and preferably between 6 and 8.

BHET in the form of crystals is recovered by filtering and then dried.

According to method (B), the reactor is loaded with waste PET, EG (some coming from internal recycling and some as a virgin monomer) and the catalyzer.

This first stage of the reaction is carried out at a temperature ranging from 180 to 270°C, and preferably between 190 and 210°C.

At the end of the first stage, the reaction mix is first filtered to eliminate any coarse impurities, then cooled by adding water to 60-90°C, and preferably 65-75°C. The amount of water added, which may affect the ease with which the subsequent stages of the process occur and the overall cost of the BHET, may vary with the reaction mix in a ratio of 0.1 to 10 (mass/ mass) and preferably between 0.5-2. The temperature control during this phase is particularly critical since the BHET must solubilize in the aqueous solution, while the oligomers (which otherwise co-crystallise with the BHET, preventing the formation of sufficiently large BHET crystals and contaminating it), must remain in suspension (see example 4).

After filtering, with separation of the oligomers, the aqueous solution is cooled slowly to precipitate out the BHET, the water solubility of which varies with the temperature as shown in graph 1 (see example 5). To obtain a high yield of BHET, the final temperature must reach -10/+30°C, and preferably 5-15°C. The BHET crystals are separated from the aqueous solution, which contains most of the excess EG, through filtering or centrifuging.

The solid recovered is dissolved again in hot water until reaching a temperature of 70-100°C and then cooled to 0-30°C, and preferably 5-15°C, to obtain high-purity BHET crystals that are easy to filter. In this second crystallisation phase, the ratio of the amount of water to BHET ranges between 4 and 10, and preferably between 6 and 8.

BHET in the form of crystals is recovered by filtering.

According to method (C), after completing the reaction similarly to what was described for method A and B, and after filtering any coarse impurities, water is added to the reaction mix in an amount that may vary in a ratio ranging from 0.1 to 10 (mass/ mass) and preferably 0.5-2 and cooled to -10/+30°C, and preferably 5-15°C. After separation through filtering or centrifuging, hot water is added to the filtered solid in an amount that may vary in a ratio ranging from 0.1 to 10 (mass/mass), and preferably 0.5-2, up to 60-90°C, and preferably 65-75°C. The temperature control during this phase is particularly critical since the BHET must solubilize in the aqueous solution, while the oligomers (which otherwise co-crystallise with the BHET, preventing the formation of sufficiently large BHET crystals and contaminating it), must remain in suspension (see example 4).

After filtering, with separation of the oligomers, the aqueous solution is cooled slowly to precipitate the BHET, the water solubility of which varies according to the temperature as shown in graph 1 (see example 5). To obtain a high yield of BHET, the final temperature must reach -10/+30°C, and preferably 5-15°C. The BHET crystals are separated from the aqueous solution, which contains most of the excess EG, through filtering or centrifuging.

The solid recovered is dissolved again in hot water until reaching a temperature of 70-100°C and then cooled to 0-30°C, and preferably 5-15°C, to obtain high-purity BHET crystals that are easy to filter. In this second crystallisation phase, the ratio of the amount of water to BHET ranges between 4 and 10, and preferably between 6 and 8.

BHET in the form of crystals is recovered by filtering.

In all three of the proposed methods, the aqueous solutions obtained from the various separation operations can be used, through distillation, to separate water and EG which can be recycled in the various phases of the process. The oligomers obtained as a residue in the second filtering of the aqueous solution, are also recycled by adding them to PET and EG in the reactor of the first stage of the process.

The heavy metal content of the BHET obtained from the process described in this invention was measured by means of an atomic absorption analysis (example 1, table 2).

The BHET obtained from this process, with method A or with method B and C, was used to prepare new PET using only Sb₂O₃ (0.8 g/Kg BHET) as the catalyzer. The PET obtained has the characteristics reported in example 6 (table 3).

A similar process was previously proposed by other authors (Indian Patent 143323, 1977); however, in the process proposed in that patent, the BHET is subjected to a recovery treatment that differs from what is proposed in this invention and such a process does not recycle the solutions recovered after filtering or centrifuging.

In particular, the reaction mass is cooled rapidly at a temperature of 90-100°C, and then slowly to ambient temperature without any filtering. As verified, that type of procedure, compared to the conditions proposed in this paper, involves a more difficult separation of the BHET during crystallisation and problems during the filtering phase since the solid is separated as a fine powder. The procedure also leads to a significant reduction in BHET yield due to its relatively high solubility at ambient temperature (a quantity of BHET that in addition to reducing the yield, increases the fraction of by-products that must be eliminated separately) and, in particular, produces BHET with lower purity since it is contaminated by a fraction of oligomers. This contamination is demonstrated by the value of the melting temperature of the product described as BHET in the aforementioned patent (110-111°C). Such a value is lower than that for the BHET obtained with the process described in this invention (116°C).

The following examples are reported to demonstrate the referenced invention and are not restrictive in any way like the claims.

### Example 1, method A

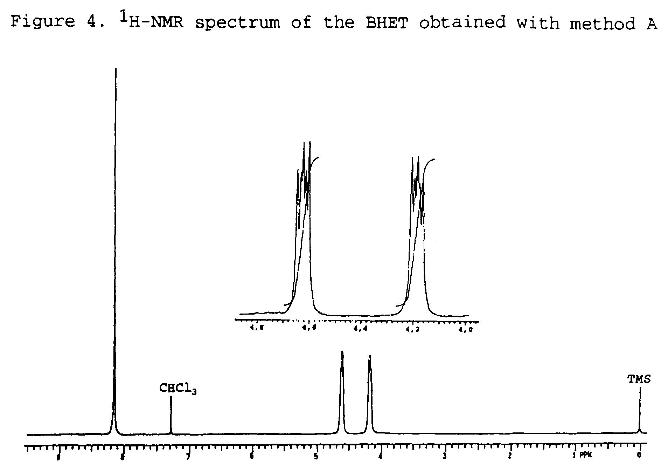

Procedure: 300 g of polyethylene terephthalate in scales and 0.45 g of ZnAc₂ were refluxed with 600 g of EG at atmospheric pressure for 2 hours. At the end of this period, the solution was hot filtered and then allowed to cool to ambient temperature. After cooling, the reaction mix, in the form of a paste, was filtered. 615 g were obtained from this second filtering phase, consisting mainly of BHET, EG and oligomers. After adding 1000 g of hot water to this product of the second filtering operation, the solution was filtered at a temperature of 70°C obtaining 42 g of a solid consisting mainly of oligomers. The filtered solution, obtained in this third filtering operation, was cooled to a temperature of 15°C and then filtered. A residue of 440 g was obtained consisting mainly of BHET and containing also water and EG. 1.5 litres of hot water was added to this product up to a temperature of 80°C. Aciculiform crystals of BHET formed while the solution cooled, which can be separated by filtering. 304 g of BHET were obtained by filtering the solution at a temperature of 15°C. The characteristics of the BHET are summarised in table 2 and the relative ¹H-NMR spectrum is reported in figure 4.

**Table 2.**

| Chemical-physical characteristics of the BHET obtained with method A: | |
|---|---|
| Colour | white |
| melting point | 116 °C |
| crystalline shape | aciculiform |
| metal content: | |
| Ca | 5 ppm |
| Zn | 6 ppm |
| Mn | 2 ppm |
| Co | none |
| Ti | none |

### Example 2, method B

Procedure: After completing the depolymerisation reaction using the same quantities and the same reaction conditions as already described in method A, the solution was hot filtered and then 1000 g of water were added. The solution was then filtered at a temperature of 70°C obtaining 45 g of a solid consisting mainly of oligomers. The filtered solution, obtained in this second filtering operation, was cooled to a temperature of 15°C and then filtered. A residue of 485 g was obtained consisting mainly of BHET and containing also water and EG. 1500 g of hot water were added to this residue up to a temperature of 80°C. Aciculiform crystals of BHET formed while the solution cooled, which can be separated by filtering. 295 g of BHET were obtained by filtering the solution at a temperature of 15°C. The characteristics of the BHET are absolutely comparable with those reported for the product obtained with method A.

### Example 3, Method C

Procedure: After completing the depolymerisation reaction using the same quantities and the same reaction conditions as already described in method A, the solution was hot filtered and then 1000 g of water were added. The solution was allowed to cool to a temperature of 15°C and then filtered. A residue of 595 g was obtained consisting mainly of BHET and oligomers and containing also water and EG. After adding 1000 g of hot water to the residue of the second filtering operation, the solution was filtered at a temperature of 70°C, obtaining 44 g of a solid consisting mainly of oligomers. The filtered solution was then cooled to a temperature of 15°C and then filtered again. A residue of 435 g was obtained consisting mainly of BHET and containing also water and EG. 1500 g of hot water were added to this residue up to a temperature of 80°C. Needle-shaped crystals of BHET formed while the solution cooled, which can be separated by filtering. 301 g of BHET were obtained by filtering the solution at a temperature of 15°C. The characteristics of the BHET are absolutely comparable with those reported for the product obtained with method A and B.

Example 4, BHET crystallisation tests with and without oligomers Using a temperature control batch reactor with a capacity of two litres, equipped with a mechanical mixer, it was observed how the solubility of BHET and the respective oligomers in aqueous solutions varies in relation to temperature. The tests were performed with (BHET+oligomers):water ponderal ratios of, respectively, 1:10 and 1:5. In particular, 1000 g of H₂O and respectively 100 and 200 g of a solid, for which 90% of the weight is BHET and 10% oligomers, were placed in the reactor at an initial temperature of 100°C. The temperature of the solution was then decreased gradually, analysing the composition of the solid phase present at various temperatures. In all the tests carried out it was found that the superior oligomers of the PET were insoluble also at temperatures close to the boiling temperature of the solution. The oligomers with a lower molecular weight (dimers and trimers) were soluble up to a temperature of 80-90°C, starting to precipitate at lower temperatures, while the BHET is soluble at these compositions in water, for temperatures higher than 60°C. During these tests it was also noted that BHET crystallisation in the presence of a solid phase, consisting of oligomers, led to the formation of irregular and small-sized crystals. Subsequent crystallisation tests only on BHET in water, using the same ratios as in the previous tests, indicated instead the formation of regular and large-sized BHET crystals.

### Example 5, BHET water solubility

To evaluate BHET water solubility in relation to the temperature of the solution, a series of tests was performed using a temperature control batch reactor with a capacity of two litres, equipped with a mechanical mixer, and starting from a solution consisting of BHET and water in a ponderal ratio of respectively 1:10. In particular, 1000 g of H₂O and 100 g of BHET and 10% oligomers, were placed in the reactor at an initial temperature of 70°C (temperature at which the BHET is completely solubilised). Then the temperature of the solution was decreased gradually causing the BHET to crystallise in solution. After having left the solution at the temperature being examined for about 3 h, some samples were taken only of the aqueous phase, which produced the experimental results reported in graph 1. Additional samples taken after much longer time intervals (up to 20 h), did not produce substantially different results.

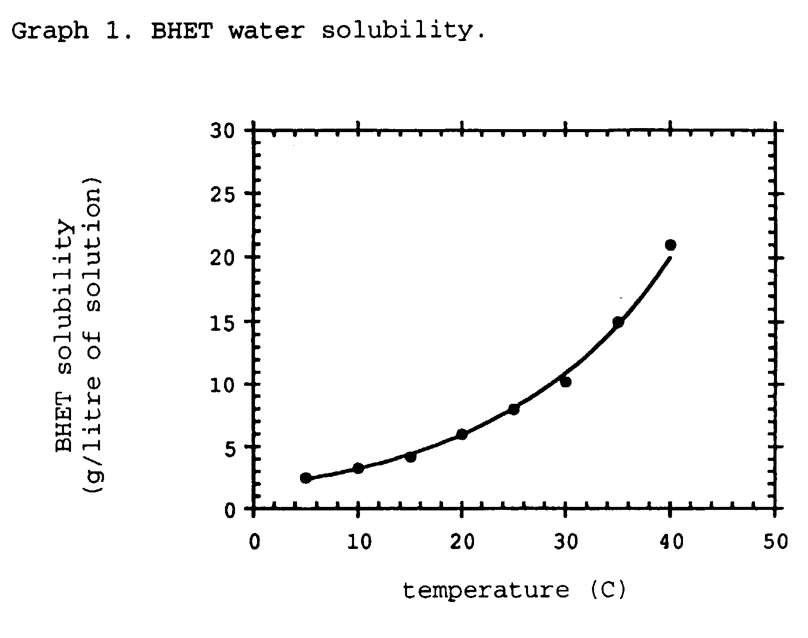

### Example 6, PET polymerisation of the BHET obtained with method A.

The BHET obtained according to procedures A, B and C as described in this paper, was then PET polymerised. 400 grams of BHET and 0.20 g of Sb₂O₃ were added to a discontinuous steel reactor equipped with a mechanical mixer. The reaction mix was then raised to a temperature of 275°C at which it reacted for 2 hours at a pressure of 1 mbar, distilling EG. PET was obtained at the end of the polymerisation operation and its relative characteristics are reported in table 3.

**Table 3.**

| Chemical-physical characteristics of the PET obtained from the polymerisation of BHET: | |
|---|---|
| Colour | colourless |
| intrinsic viscosity⁽¹⁾ | 0.90 dl/g |
| diethylene glycol content: | 0.4% |
| carboxyl terminal groups | 18 meq./kg |
| melting point⁽²⁾ | 255°C |
| crystallisation heat⁽²⁾ | -38 J/g |
| metal content: | < 15 ppm |

| | |
|---|---|
| (1) At a temperature of 30°C, using a mix of phenol/ tetrachloroethane 40/60 (m/m) as a solvent | |
| (2) Determined by means of DSC, scanning speed 20°C/min. | |

## Claims

1. Process to prepare BHET in which high-purity BHET can be obtained through the reaction of waste PET with excess ethylene glycol and with which pure BHET can be recovered through controlled crystallisation processes from an aqueous solution. The depolymerisation reaction occurs in the presence of a transesterification catalyser, such as Zn, Ca or Co acetates, at a temperature of 190-210°C, for a period of 2-3 hours. After cooling the reaction mix, a solid consisting of BHET and oligomers can be separated by filtering or centrifuging from most of the excess glycol; subsequent solubilisation of the solid in water, followed by precipitation, crystallisation and filtering (or centrifuging) at specific temperatures, are used to obtain BHET with high purity and good yields.

2. Process as described in claim 1 in which the weight ratio between EG and PET ranges between 1 and 5.

3. Process as described in claim 1 in which the PET and any EG are added in the reactor that already contains a predetermined quantity of reaction mix.

4. Process as described in claim 1 in which water is added to the reaction product, the temperature of which is increased to 65-75°C and which is then filtered (or centrifuged) to separate all the oligomers present.

5. Process as described in claim 1 in which water is added to the reaction product and filtering (or centrifuging) is performed at a temperature of 5-15°C to separate part of the EG from the mix of oligomers followed by recovery of pure BHET.

6. Process as described in claim 1 in which the overall yield of the final BHET obtained (considering the direct recycling of the separated oligomers) is greater than 90% compared to the initial PET.

7. Process as described in claim 1 in which the BHET obtained is so pure that it can also be used to produce PET for food applications.
